# EUROPEAN PATENT SPECIFICATION

(11) **EP 3 582 822 B1**
(45) Date of publication and mention of the grant of the patent: **18.12.2024**
(21) Application number: 18813474.6
(22) Date of filing: 12.02.2018
(51) Int. Cl.: A61M 1/14

(54) **A VENOVENOUS ECMO (EXTRACORPOREAL MEMBRANE OXYGENATION) CANNULA**
VENOVENÖSE ECMO (EXTRAKORPORALE MEMBRANOXYGENIERUNGS)-KANÜLE
CANULE (D'OXYGÉNATION PAR MEMBRANE EXTRACORPORELLE) ECMO VEINO-VEINEUSE

(30) Priority: 15.02.2017 TR 201702278
(43) Date of publication of application: 25.12.2019
(73) Proprietor: T.C Medipol Üniversitesi, Istanbul (TR)
(72) Inventor: SALIHOGLU, Ece, Bagcilar/Istanbul (TR); YILDIZ, Yahya, Bagcilar/Istanbul (TR)
(74) Representative: Simsek, Meliha Merve
(86) International application number: PCT/TR2018/000010
(87) International publication number: WO 2018/226187

(56) References cited:
- WO-A1-2015/089047
- WO-A1-2015/089047
- WO-A1-2015/199486
- WO-A1-97/37718
- WO-A2-02/098499
- WO-A2-99/58174
- US-A1- 2001 016 729
- US-A1- 2002 128 586
- US-A1- 2003 093 027
- US-B1- 6 264 645

## Description

### Technical Field

This invention is related to a dual lumen triple brimmed venovenous ECMO (Extracorporeal Membrane Oxygenation) cannula (1)

### Prior Art

Extracorporeal Membrane Oxygenation (ECMO) is a method used in patients with heart or respiratory failure in all age groups who do not respond to traditional treatment modalities. With the ECMO system, cannula or cannulas are used to remove dirty (venous) blood, and after the oxygenation of the blood is established, the arterial blood is re-inj ected.

Especially in pediatric age groups, cannulas used for venovenous (VV) ECMO, may have performance problems due to inadequacy of blood collection and blood supply capacity. To overcome this problem, it is necessary to use larger cannulas according to patient sizes/dimensions. As a result, there is a risk of discomfort during insertion and damage to the patient's blood vessels or heart and surrounding organs during application. In addition, an echocardiography device is used to detect the position of the cannula which was difficult to place, and the use of this device for this purpose also requires special experience. When the appropriate position is obtained in the cannula, the cannula may come off from the desired position with a small movement of the patient. In addition, cannulas that are placed in to that patients that stay in the hospital for a long time pose a risk for infection The difficulties mentioned above limit the use of ECMO in the appropriate patients who need them, reduce the benefit of ECMO treatment, prolong hospitalization time, increase hospital deaths, increase patient mortality and increase health expenditures.

The United States patent no. US2005085761, which is incorporated in the prior art, refers to a double lumen cannula expandable for venous ECMO. This document discloses an apparatus, system and method for the use of a simple, less invasive, self-expanding, intradermal double lumen cannula group for VV ECMO. The invention described in this document provides theoretical total venous blood drainage, total extracorporeal gas exchange, and prevent recirculation and more than one (multiple) cannula use. Thus, VV ECMO is simplified, surgery and blood trauma are diminishing and its application is expanding. The cannula described in the document has three apertures in one embodiment. The dual lumen cannula of the invention also has a radiopaque marker. The cannula described in the United States patent document has a rigid structure and also has an adjustable flow mechanism. However, since this design does not include a design variety that adapts to changes in size and anatomical structure in accordance with the development of the patient, the performance of the cannula may be uncertain or under ideal. In the same design, the upper suction mouth is located very close to the spray mouth, which can cause the arterial (clean) blood to pass back through the venous (dirty) blood suction holes in the system (ie some sort of short circuit mechanism - recirculation). This can cause the total amount of oxygenated blood to be low. There is no fixing element in this design, which carries the risk of moving of cannula from where it was fixed to the skin with simple seam.

There is no structural member to prevent narrowing of the inner lumen via bowing and twisting of the cannula described in the United States patent document US2005085761. In the same design, the cannula is circular in cross-section and the structure separating the suction and ejection channels is flexible. With this design, varying lumen diameters in accordance with suction-spray power can be risky for flow stabilization. This cannula design does not have a special construction element resistant to infection. In this design, there is only one indicator showing the position of the cannula in the heart and vein. While this shows this level of cannula, it is insufficient to show the direction of openness (port).

A coaxial venous cannula is disclosed in the United States patent document US9233223, which is another prior art document The double lumen cannula described in the document comprises two intertwined lumens wherein it has a proximal end, a distal end .In said cannula, while the first tube terminates with a multi-hole proximal end there is a second tube concentric with the first tube and continuing constantly in the middle, and there is also a distal end terminating with a drain hole therefrom.

Additionally, in the said document the location of the lumen is coaxial in the mentioned cannula and the circular structure of the cannula restricts the amount of blood that is absorbed and ejected.

In said cannula, the openings in the cannula are of a very porous structure, and it was designed this way so that every opening can perform both the suction and, the depending on the application position, can function to eject from both the neck and the head. However, this structure does not take into account the fact that the suction and ejection forces are countercurrent, so that the blood cells have a risk of causing damage during the process.

It is doubtful that placing openings at only two levels in the cannula would provide adequate blood sucking and pressure, and also it seems inevitable that this structure will be insufficient to collect venous blood from the upper and lower body of the circulatory system and direct them to a valve in a middle level. Again, in this design it is inevitable that there will be an increased short circuit - recirculation amount, which means that the performance of the cannula will be low. Also, the length of the cannula was kept long so that it can also be applied from the leg of a patient. However, this makes the use of this cannula inappropriate for short patients. For this cannula pulmonary artery placement is also recommended, however this method of placement is highly invasive (has high risk of damage to tissues) , its benefit is limited and doubtful. Moreover, this practice increases the cost because it requires special experience and special equipment (such as an angio laboratory or advanced echocardiographic imaging). Again, in this type of application, a too long cannula in the heart affects the proper functioning of the heart. There is no stabilizing structure element in the same design. For this reason, there is a risk of protecting the position of the cannula. This cannula design also lacks a marker structure that indicates the position of the cannula, so a special imaging experience (echocardiography / ultrasound) is required to ensure that the cannula is correctly positioned.

Other prior art documents that are of relevance to the present invention and their brief disclosures are given below.

WO2015199486 discloses an integrated double cannula for ECMO. Said cannula comprises a cannula body constituted by integrally forming a blood introduction part and a blood supply part that are separated from each other by a partition wall in the interior of a single pipe. Said cannula body also has an end hole and also an inlet and an outlet that are open side by side. The cannula body comprises multiple side holes that allows introduction of a large amount of blood into ECMO.

WO02098499 discloses a multilumen catheter that is designed for maximizing the bloodflow into and out of the patient's vasculature while providing for passive or active perfusion of tissue downstream of where the catheter resides. The catheter of the document comprises a first and a second distal end and the luments extending between each of these distal ends and the proximal end provide for blood circulation.

WO9958174 discloses a circulatory support system comprising at least one venous cannula, at least one arterial cannula and at least one blood circulation pump connected between these two cannulae. This way an isolated circulatory loop is formed to support the circulatory system of a patient.

US2003093027 discloses a catheter omprising a catheter body having a distal portion, a central return lumen extending from a proximal portion of the catheter body to the distal portion to allow blood passage therethrough, at least three longitudinally extending intake lumens independent of the return lumen and radially displaced with respect to the return lumen, and means for enabling simultaneous cut off of fluid flow to the at least three longitudinally extending lumens.

US6264645 discloses a multi lumen cannula comprising a first sub-cannula and a second sub-cannula. The first sub-cannula includes a proximal end, a distal end, a lumen extending between the proximal and distal ends, and proximal and distal fluid apertures formed in the lumen. The second sub-cannula includes a proximal end, a distal end, a lumen extending between the proximal and distal ends, and proximal and distal fluid apertures formed in the lumen. The distal fluid apertures of the two lumens are spaced from one another along the axial length of the multi-lumen cannula.

WO9737718 discloses a dual lumen catheter that allows access to the vascular system of a human for providing high volume fluid flow. The crosssectional area of the catheter is designed to be minimal for maintaining the desired flow rate. The catheter comprises two tubular structures separated by a wall wherein one of the tubular structures is named as arterial line and the other is named as the venous line.

### Brief Description of the Invention

The purpose of this invention is to provide a venovenous ECMO cannula that achieves performance gains by providing higher blood flow in smaller sizes and saves practitioners from the age, weight and length constraints of patients.

Another object of the invention is to achieve a venovenous ECMO cannula that facilitates the use of venovenous ECMO and reduces infant mortality in patient groups whose weight are under ten kilograms.

The other purpose of the invention is to realize a venovenous ECMO cannula which can be placed easily and correctly and the ideal settlement position is easily detected without requiring any special experience. This purpose is achieved by means of a cannula according to claim 1. Further objects are achieved by the dependent claims.

Another object of the invention is to realize a venovenous ECMO cannula in which loss of position of the inserted cannula with the movement of the patient's body is prevented with the use of an ear clamp in the ECMO cannula.

Another object of the invention is to realize a venovenous ECMO cannula wherein the infections risk arising from the prolonged stay in patient's body is reduced by use os a material such as silver or like.

Another object of the invention is to realize a venovenous ECMO cannula that is thin, soft and resilient but that is resistant to internal luminal contraction factors such as fracture and bending, such that the risk of vessel and cardiac damage during placement is reduced.

Another object of the invention is to realize a venovenous ECMO cannula which increases the amount of blood sucked with less suction pressure thanks to the ellipsoidal cross-sectional shape.

Another object of the invention is to realize an ECMO cannula that provides a controlled blood suction and ejection process to obtain the working system closest to the natural working order of human biology.

Another object of the invention is to realize a venovenous ECMO cannula in which the ejection opening is in the form of a wide mouth and surrounded by indicators wherein its direction is towards the cardiac valve through which the location of the venovenous ECMO cannula can be determined precisely and accurately.

Another object of the invention is to realize an ECMO cannula with four indicators wherein there are two indicators at the entrance, one at the blood ejection part and finally one at the bottom, whereby the ideal position of the cannula can be detected and verified as often as desired.

Another object of the invention is to implement a venovenous ECMO cannula having upper and lower region suction tips suitable for jugular application.

Another object of the invention is to realize a venovenous ECMO cannula in which different designs can be applied according to the patient size.

### Detailed Description of the Invention

To achieve the purpose of this invention, a cannula according to claim 1 is provided. "A Venovenous ECMO (Extracorporeal Membrane Oxygenation) Cannula" is shown in the enclosed drawings which disclose;
**Figure 1****.** Side view of the large sized cannula according to present invention.
**Figure 2****.** Side view of the small sized cannula according to present invention.
**Figure 3****.** A cross-sectional view of arterial and venous cannula lumens of cannulas according to present invention.
**Figure 4****.** A view of the upper end venous openings (venous blood inlet holes) in large sized cannula according to present invention.
**Figure 5****.** A cross-sectional view of the arterial and venous lumens in the cannula of the present invention at the level of the upper venous openings (venous blood entry).
**Figure 6****.** A view of the upper end venous opening (venous blood entry) in cannula of the invention that is suitable for small sized patients.
**Figure 7****.** The lumen cross-section view at the level of lower venous end opening of the cannula according to present invention.
**Figure 8****.** The detailed top view and side view of the clamp used in the cannula.

The parts in the figures are numbered individually and their correspondences are given below.
1. Cannula
2. Body
3. Coating
4. Indicator
5. Reinforced area
6. Upper venous opening
7. Arterial opening
8. Lower venous basket end
9. Basket end opening
10. Lower end hole
11. Clamp

The ECMO cannula (1) of the present invention that enables oxygenation of the venous blood and then returning said blood back to the patient, comprises the features of claim 1.

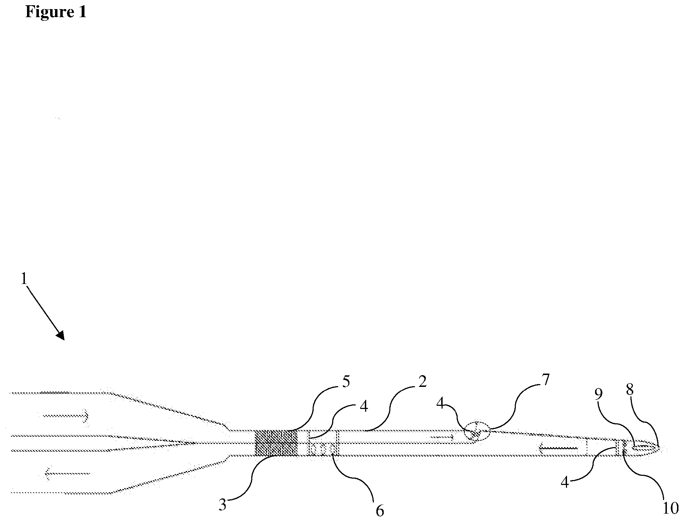

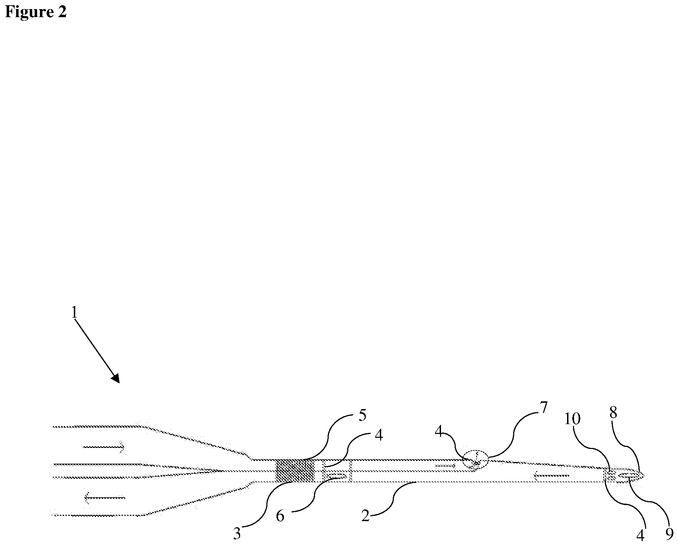

The cannula of the invention (1) comprises two ends that perform their functions separately at the upper part of the body (2) and from which the venous blood exits and is returned back to the system after cleaning. The lumens located at the body (2), which are the continuation of the ends, extend contiguously after one point and after the arterial opening (7), and transforms into a single lumen from which the blood is sucked and stretches out to the lower part of the body (2). This way the body (2) has a structure that narrows down from upper part to lower part and has a thin lower venous basket end (8). There are preferably two indicators (4) located in the upper part of the body (2) and there is upper venous openings (6) between them and the venous blood enters the cannula from these openings. The body (2) comprises two indicators (4) located on the top and bottom of the upper venous opening (6). The arterial opening (7) from which the clean blood is ejected in on the body (2), at the lower part of the indicator (4) located under the upper venous openings (6). The anti-bacterial coating (5) is located on the body (2) between the junctions of the ends and the indicator (4) located in the upper part of the venous openings (6) The reinforced area (5) preventing breakage is located between the junction of the ends and the indicator (4) located at the upper part of the upper venous openings (6) (Figure 1-2).

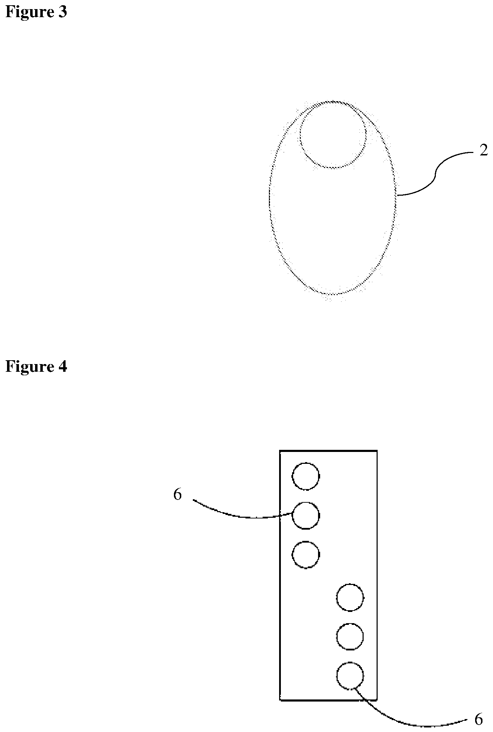

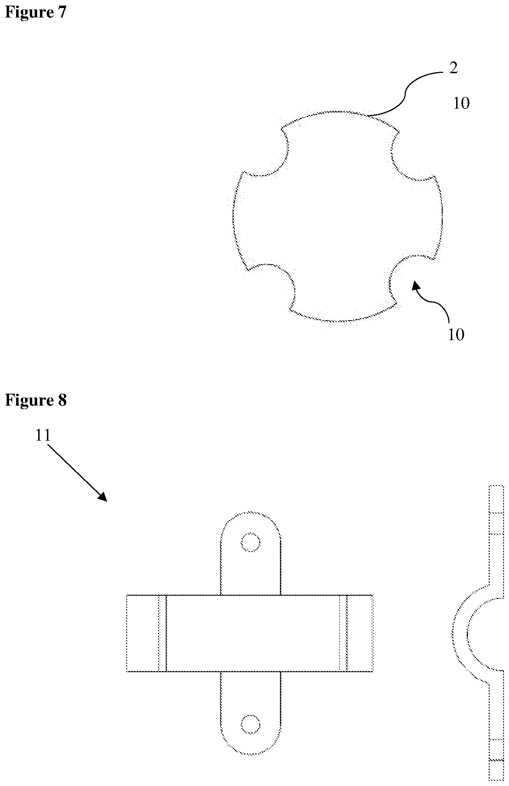

In the cannula of the present invention (1), the body (2) has an elliptical shape starting from the junction if the ends to the arterial opening (7) from which the clean blood is ejected. This leads to an increase in the surface area of the body (2) and the volume of venous lumen. A higher number of venous openings (6) that are bigger in size can be placed on the increase surface area of the body (2). The arterial lumen located in elliptical body (2) and from which the clean blood ejected has a circular section. (Figure 3) In a preferred embodiment of the invention, the distal venous ends have a circular cross-section (Figure 7) .The lumens in the body (2) are not concentric, but have a lumen with circular cross-section in which the blood to be ejected proceeds.

In the present invention, the anti-bacterial coating (3) is preferably made of silver or ta similar material . The anti-bacterial coating (3) is located on the part of the body (2) that is in contact with patient's skin preferably at the upper part of the indicator (4) on the upper part of the body (2). With the use of anti-bacterial coating (3), the risk of infecting the patient is reduced in long-term use.

According to the invention, the indicator (4) is a band made of radio opaque material In a preferred embodiment; on the body (2) there are preferably four indicators (4). Indicators (4) located at the upper part of the body (2), at the top and bottom of upper venous openings (6) indicate the beginning and end of venous openings. The indicator (4) located just above the lower venous basket end (8) at the bottom of the body (2), is the lower radiopaque indicator of the venous lumen. On the body (2), the indicator (4) located in the arterial opening (7) is C-shaped and indicates the arterial opening. Said indicator (4) is used to direct the direction of the arterial opening (7) towards the heart valve. Indicators (4) are used to place the cannula (1) according to the natural structure of human biology and to maintain this convenient and efficient location.

In the cannula according to present invention (1), the reinforced area (5) used to prevent fracture of the body (2) is stretchable but resistant to breakage. In this way, twisting of the body (2) from the reinforced area (5) is prevented and thus the constriction or closing of the inner lumen and a decrease or stopping of the blood flow is prevented.

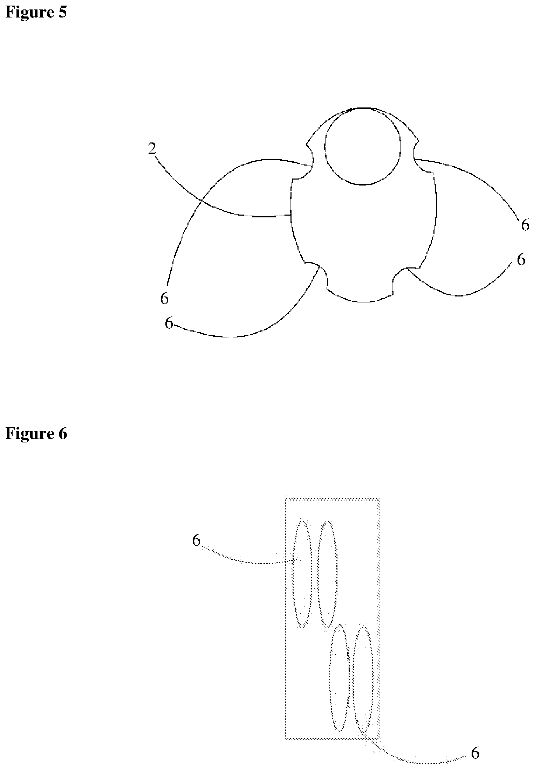

In a preferred embodiment of the invention, the upper venous opening (6) located at the top of the body (2) and used for entry of the venous blood can be in various shapes and numbers depending on the patient development. In the case where the patient size has increased, there are more than one round cross-sectioned upper venous openings (6) between the two indicators (4) located on the upper part of the body (2) (Figure 6). Said upper venous openings (6) are located gradually on the body (2) (Fig. 5). In the case where the patient size is reduced, there are elliptical cross-sectioned upper venous openings (6) between the two indicators (4) located on the upper part of the body (2) (Figure 7). Said upper venous openings (6) are elongated by extending along the body (2) and thus has an eliptical shape. This is because the amount of dirty blood coming from the upper part of the body is high because circulatory physiology changes in small-sized patients. With the design difference in small sized patients the surface area of the upper venous opening (6) is increased so that more blood is collected from the upper part of the body, thereby the performance of the cannula (1) is increased.

In the present invention, (1) the arterial opening (7) to which clean blood is ejected preferably has a round or transverse or longitudinal elliptical cross-section. In addition , the problem of hemolysis, that is, damage of blood elements by breaking, is minimized by the curved structure of the arterial opening (7) from which the blood exits .

In the cannula of the invention (1) the lower venous basket end (8) is located at the extreme end of the lower part of the body (2). On the upper part of the lower venous basket (8) there are more than one lower end holes named as lower end holes (10).In a preferred embodiment basket end openings (9) are located at the distal end of the venous lumen.

In a preferred embodiment of the invention, the number and shape of lower end hole (10) located under the body (2) and above the lower venous basket end hole (10) on the lower venous end (8) varies with respect to the size of the patient. In the case where the patient size is enlarged, there are a plurality of round or elliptic cross sectioned lower end holes (10) on top of the lower venous basket end (8) below the body (2).In the case where the patient size is smaller, there are elliptic or round shaped lower end holes (10) on top of the lower venous basket end (8) below the body (2)Said lower end holes 10 are elongated by extending along the body (2).

In the present invention, the number of the upper venous opening (6) and the lower end openings (10) used for suction is increased so as to increase the suction surface as a result which a cannula with a smaller body (2) provides more suction power and hence enhance the performance of the cannula (1). This way, the thinner cannula (1) provides better flow dynamics and facilitates ease of placement of the cannula (1). In the cannula according to invention(1) the upper venous opening (6) and lower end hole (10) are located on both sides of the body (2). This way, the amount of blood sucked and ejected into the system is kept in a way that does not disturb the body balance. With the help of the upper venous opening (6) and lower end hole (10) it is possible to withdraw blood from both the neck and the leg.

In the small cannula (1) designed according to the patient size, the ratio of the part comprising upper venous openings (6) located on the upper side of the body (2) and used for suction to the entire body of the cannula is more, whereas in big cannula (1) the suction part comprising lower end holes (10) located at the lower side of body (2) is more. The reason is in small children the ratio of upper body to lower body is in favor of upper body whereas as they grow up and take up adult sizes this ratio increases in favor of the lower body comprising the body and the legs.

In the cannula (1) of the present invention, the clamp (11) can be attached to and detached from the body (2) and with the help of two ears that are present on the clamp can be tied to the skin of the patient (11) with preferably a suture thread. The clamp (11) can be attached to and detached from the cannula (1) body (2) with the help of its opening and its elastic structure. Said clamp (11) comprises two channels that help fixing to the skin via winding of the suture. In a preferred embodiment of the invention the clamp (11) is used in the region that is between the reinforced area (5) and the lumen ends on the body (2).

In a preferred embodiment of the invention in the size of the body (2) designed according to the size of the patient, the distance between upper venous openings used for blood suction and lower end holes (6, 10) and the arterial openings (7) used for ejection of blood are kept in a way that would reduce the recirculation/shunt probability ratio. The cannula (1) of the invention the cross-sectioned body (2) constructs provide better flow performance. Additionally, with the cannula (1) the friction and turbulence is reduced and as a result the problem of damage to blood elements via fragmenting decreases. The possibility of varying the size of the cannula (1) according to the size of the patient not only provides increase in performance but also provides that the amount of blood sucked from the neck and leg of the patient is in accordance with the biology of the patient. The size of the cannula (1) can be changed according to the length / kg ratio and the size of the cannula (1) can be changed and also by creating a cannula (1) portfolio wherein the parameters such as cannula (1) length, arterial opening (7), upper venous opening (6), lower end hole (10) can be changed, the cannula (1) that is most suitable for the patient can be chosen. Furthermore, with the cannula (1) infant mortality is reduced by facilitating the use of venovenous ECMO in the patient group weighing less than 10 kilograms.

Around these basic concepts, it is possible to develop a wide variety of applications for the inventive subject the invention "A venovenous ECMO (Extra corporeal Membrane Oxygenation) cannula (1)", and the invention is not limited to the examples disclosed herein, but is as specified in the claims.

## Claims

1. A cannula (1) for the oxygenation of contaminated blood and for returning the blood to a patient, said cannula (1) comprising;
- at least one body (2) having a plurality of openings in which, in use, the blood is collected and sprayed, the body having a double lumen structure with ends from which, in use, dirty blood leaves the body (2) and the clean blood enters the body (2),
- at least one anti-bacterial coating (3) located on an upper part of the body (2) that is configured to be in contact with the patient's skin,
- more than one indicator (4), each indicator (4) being a band made of radio opaque material that determines the level and one indicator (4) being located on the upper part of the body (2) in an arterial opening (7) at which clean blood is to be ejected and a further indicator being located at a the lower part of the body (2),
- at least one reinforced area (5) that is resilient and resistant to breakage and that is located in the upper part of the body (2),
- at least one upper venous opening (6) located on the body (2) and configured for the entry of venous blood,
- at least one lower venous basket end (8) located at a lower end of the body (2) and having at least one basket end opening (9),
- at least one lower end hole (10) on the body (2) for the withdrawal of blood, which is located such that it is on the upper side of the lower venous basket end (8),
- at least one clamp (11) configured for securing the body (2) on the patient's skin,
- the body (2) being elliptical in shape in order to increase the amount of blood sucked, the body being formed by the non-concentric placement, within the elliptical body (2), of the lumen that is used to inject blood, the said lumen that is used to inject blood having a circular cross section.

2. A cannula (1) according to claim 1, **characterized in that** said body (2) comprises two ends connected with lumens and wherein said ends perform their functions separately at the upper part of the body (2) and from which the venous blood exits and is returned back to the system after cleaning.

3. A cannula (1) according to claim 1 or 2 **characterized in that** said body (2) comprises two indicators (4) on the upper region and there are upper venous openings (6) in between the indicators from which the dirty blood enters.

4. A cannula (1) according to any of the preceding claims **characterized in that** said body (2) comprises two indicators (4) located at the upper and lower part of the upper venous openings (6).

5. A cannula (1) according to any of the preceding claims **characterized in that** said arterial opening (7) from which the clean blood is ejected is located on the body (2) under the indicator (4) located under the upper venous opening (6).

6. A cannula (1) as in any of the preceding claims, **characterized by** :
- indicators (4) located at the top and bottom of the upper venous openings (6) at the top of the body (2), indicating the beginning and end of openings, and/or
- an indicator (4) just above the lower venous basket end (8) at the bottom of the body (2), that is the lower radiopaque indicator of the venous lumen , and/or
- an indicator (4) that is C shaped and is located on the opening (7) on the body (2) defining the arterial opening .

7. A cannula (1) according to any of the preceding claims **characterized in that** said upper venous opening (6) that is located on the top of the body (2) is configured such that its shape and number varies with respect to the size of the patient and is used for introduction of contaminated blood.

8. A cannula according to claim 7, **characterized in that** said cannula (1) comprises more than one round cross-sectioned upper venous openings (6) between the two indicators (4) located on the upper part of the body (2) when the patient size is increased.

9. A cannula (1) according to claim 7 or 8, **characterized in that** said cannula (1) comprises upper venous openings (6) which are located gradually on the body (2).

10. A cannula (1) according to claim 7, **characterized in that** said cannula (1) comprises elliptical cross-sectioned upper venous openings between the two indicators (4) located on the upper part of the body where the patient size is reduced.

11. A cannula according to any of the preceding claims, **characterized in that** said arterial opening (7) has a round or transverse or longitudinal elliptical cross-section and to which clean blood is ejected.

12. A cannula (1) according to any of the preceding claims **characterized in that** said lower venous basket end (8) has more than one hole, named as lower end holes (10) on top of it.

13. A cannula (1) according to claim 12, **characterized in that** said cannula comprises basket end openings (9) located on the distal end of the venous lumen.

14. A cannula (1) according to any of the preceding claims wherein said lower end hole (10) located under the body (2) and above the lower venous end (8) is configured such that its number and shape varies with respect to the size of the patient such that;
- where the patient size is enlarged, there are a plurality of round or elliptic cross sectioned lower end holes (10) on top of the lower venous basket end (8) below the body (2), or
- where the patient size is smaller, there are elliptic or round shaped lower end holes (10) on top of the lower venous basket end (8) below the body (2) .

15. A cannula (1) according to any of the preceding claims **characterized in that** said upper venous opening (6) and said lower end hole (10) are located on both sides of the body (2) in order to provide surface increase for blood suction so that there will be more suction power with a narrow body (2).

## Patentansprüche

1. Kanüle (1) zur Sauerstoffversorgung von verunreinigtem Blut und zur Rückführung des Blutes zu einem Patienten, wobei die Kanüle (1) umfasst;
- mindestens einen Körper (2) mit einer Vielzahl von Öffnungen, in denen bei der Verwendung das Blut gesammelt und versprüht wird, der Körper eine doppelte Lumen Struktur mit Enden aufweist, aus denen bei der Verwendung schmutziges Blut den Körper (2) verlässt und sauberes Blut in den Körper (2) eintritt,
- mindestens eine antibakterielle Beschichtung (3), die sich auf einem oberen Teil des Körpers (2) befindet, der konfiguriert ist, um mit der Haut des Patienten in Kontakt zu sein,
- mehr als einen Indikator (4), jeder Indikator (4) ein Band aus funkdurchlässigem Material ist, das den Füllstand bestimmt, und ein Indikator (4) am oberen Teil des Körpers (2) in einer arteriellen Öffnung (7) angeordnet ist, an dem sauberen Blut ausgestoßen werden soll, und ein weiterer Indikator am unteren Teil des Körpers (2) angeordnet ist,
- mindestens einen verstärkten Bereich (5), der elastisch und bruchfest ist und sich im oberen Teil des Körpers (2) befindet,
- mindestens eine obere venöse Öffnung (6), die sich am Körper (2) befindet und für den Eintritt von venösem Blut konfiguriert ist,
- mindestens ein unteres venöses Korbende (8), das sich an einem unteren Ende des Körpers (2) befindet und mit mindestens einer Korbendöffnung (9),
- mindestens ein unteres Endlöcher (10) am Körper (2) für die Entnahme von Blut, das so angeordnet ist, dass es sich auf der Oberseite des unteren venösen Korbendes (8) befindet,
- mindestens eine Klammer (11), die zum Befestigen des Körpers (2) an der Haut des Patienten ausgebildet ist,
- der Körper (2) eine elliptische Form hat, um die Menge des angesaugten Blutes zu erhöhen, der Körper durch die nicht-konzentrische Anordnung des Lumens, das zur Injektion von Blut dient, im Inneren des elliptischen Körpers (2) gebildet wird, das Lumen, das zur Injektion von Blut dient, einen kreisförmigen Querschnitt aufweist.

2. Kanüle (1) nach Anspruch 1,
**dadurch gekennzeichnet, dass**
der Körper (2) umfasst zwei Enden, die mit Lumen verbunden sind, wobei die Enden ihre Funktionen getrennt im oberen Teil des Körpers (2) ausüben und aus dem das venöse Blut austritt und nach der Reinigung wieder in das System zurückgeführt wird.

3. Kanüle (1) nach Anspruch 1 oder 2,
**dadurch gekennzeichnet, dass**
der Körper (2) im oberen Bereich zwei Indikatoren (4) aufweist und zwischen den Indikatoren obere venöse Öffnung (6) vorhanden sind, aus denen das verschmutzte Blut austritt.

4. Kanüle (1) nach einem der vorhergehenden Ansprüche,
**dadurch gekennzeichnet, dass**
der Körper (2) umfasst zwei Indikatoren (4), die sich am oberen und unteren Teil der oberen venöse Öffnung (6) befinden.

5. Kanüle (1) nach einem der vorhergehenden Ansprüche,
**dadurch gekennzeichnet, dass**
die arterielle Öffnung (7), aus der das saubere Blut ausgestoßen wird, befindet sich am Körper (2) unter dem Indikator (4), der sich unter der oberen venösen Öffnung (6) befindet.

6. Kanüle (1) nach einem der vorhergehenden Ansprüche, im Folgenden **gekennzeichnet durch**:
- Indikatoren (4), die sich oben und unten an den oberen venösen Öffnungen (6) oben am Körper (2) befinden, den Beginn und das Ende von Öffnungen anzeigt, und/oder
- ein Indikator (4) direkt über dem unteren venösen Korbende (8) am Boden des Körpers (2), der den unteren röntgendichten Indikator des venösen Lumens und/oder
- ein Indikator (4) die C-förmig ist und sich an der Öffnung (7) am Körper (2), die arterielle Öffnung definiert.

7. Kanüle (1) nach einem der vorhergehenden Ansprüche,
**dadurch gekennzeichnet, dass**
die obere venöse Öffnung (6), die sich auf der Oberseite des Körpers (2) befindet, so konfiguriert ist, dass ihre Form und Anzahl in Bezug auf die Größe des Patienten variiert und zum Einführen von kontaminiertem Blut verwendet wird.

8. Kanüle nach Anspruch 7,
**dadurch gekennzeichnet, dass**
die Kanüle (1) mehr als eine obere venöse Öffnung (6) mit rundem Querschnitt zwischen den beiden Indikatoren (4) umfasst, die sich am oberen Teil des Körpers (2) befinden, wenn die Größe des Patienten vergrößert wird.

9. Kanüle (1) nach Anspruch 7 oder 8,
**dadurch gekennzeichnet, dass**
die Kanüle (1) obere venöse Öffnungen (6) aufweist, die sich allmählich am Körper (2) befinden.

10. Kanüle (1) nach Anspruch 7,
**dadurch gekennzeichnet, dass**
die Kanüle (1) elliptische, im Querschnitt obere venöse Öffnungen zwischen den beiden Indikatoren (4) umfasst, die sich auf dem oberen Teil des Körpers befinden, wo die Größe des Patienten reduziert ist.

11. Kanüle nach einem der vorhergehenden Ansprüche,
**dadurch gekennzeichnet, dass**
die arterielle Öffnung (7) hat einen runden oder transversalen oder longitudinalen elliptischen Querschnitt und auf dem sauberen Blut ausgestoßen wird.

12. Kanüle (1) nach einem der vorhergehenden Ansprüche,
**dadurch gekennzeichnet, dass**
die untere venöse Korbende (8) weist mehr als ein Loch auf, das als untere Endlöcher (10) bezeichnet wird.

13. Kanüle (1) nach Anspruch 12,
**dadurch gekennzeichnet, dass**
die Kanüle weist Korbendöffnungen (9) auf, die sich am distalen Ende des venösen Lumens befinden.

14. Kanüle (1) nach einem der vorhergehenden Ansprüche, wobei das untere Endlöcher (10), das sich unter dem Körper (2) und über dem unteren venösen Ende (8) befindet, so konfiguriert ist, dass seine Anzahl und Form in Bezug auf die Größe des Patienten variiert, so dass;
- wenn die Patientengröße vergrößert ist, gibt es eine Vielzahl von runden oder elliptischen im Querschnitt unteren Endlöchern (10) oben auf dem unteren venösen Korbende (8) unter dem Körper (2), oder
- wenn die Patientengröße kleiner ist, befinden sich elliptische oder runde untere Endlöcher (10) oben auf dem unteren venösen Korbende (8) unter dem Körper (2).

15. Kanüle (1) nach einem der vorhergehenden Ansprüche,
**dadurch gekennzeichnet, dass**
die obere venöse Öffnung (6) und das untere Endlöcher (10) auf beiden Seiten des Körpers (2) angeordnet sind, um eine Oberflächenvergrößerung für die Blutabsaugung bereitzustellen, so dass bei einem schmalen Körper (2) mehr Saugkraft vorhanden ist.

## Revendications

1. Canule (1) pour l'oxygénation de sang contaminé et pour le retour du sang à un patient, ladite canule (1) comprenant ;
- au moins un corps (2) ayant une pluralité d'ouvertures dans lesquelles, en cours d'utilisation, le sang est collecté et pulvérisé, le corps ayant une structure à double lumière avec des extrémités à partir desquelles, en cours d'utilisation, le sang sale quitte le corps (2) et le sang propre entre dans le corps (2),
- au moins un revêtement antibactérien (3) situé sur une partie supérieure du corps (2) qui est configurée pour être en contact avec la peau du patient,
- plus d'un indicateur (4), chaque indicateur (4) étant une bande faite d'un matériau radio-opaque qui détermine le niveau et un indicateur (4) étant situé sur la partie supérieure du corps (2) dans une ouverture artérielle (7) à laquelle le sang propre doit être éjecté et un autre indicateur étant situé sur la partie inférieure du corps (2),
- au moins une zone renforcée (5) qui est résiliente et résistante à la rupture et qui est située dans la partie supérieure du corps (2),
- au moins une ouverture veineuse supérieure (6) située sur le corps (2) et configurée pour l'entrée du sang veineux,
- au moins une extrémité inférieure de panier veineux (8) située à une extrémité inférieure du corps (2) et ayant au moins une ouverture d'extrémité de panier (9),
- au moins un trou d'extrémité inférieur (10) sur le corps (2) pour le retrait du sang, qui est situé de telle sorte qu'il est sur le côté supérieur de l'extrémité inférieure de panier veineux (8),
- au moins une pince (11) configurée pour fixer le corps (2) sur la peau du patient,
- le corps (2) étant de forme elliptique afin d'augmenter la quantité de sang aspiré, le corps étant formé par la mise en place non concentrique, à l'intérieur du corps elliptique (2), de la lumière qui est utilisée pour injecter le sang, ladite lumière qui est utilisée pour injecter le sang ayant une section transversale circulaire.

2. Canule (1) selon la revendication 1, **caractérisée en ce que** ledit corps (2) comprend deux extrémités connectées avec des lumières et dans laquelle ces extrémités remplissent leurs fonctions séparément à la partie supérieure du corps (2) et à partir de laquelle le sang veineux sort et est renvoyé dans le système après le nettoyage.

3. Canule (1) selon la revendication 1 ou 2, **caractérisée en ce que** ledit corps (2) comprend deux indicateurs (4) sur la région supérieure et il y a des ouvertures veineuses supérieures (6) entre les indicateurs par lesquelles le sang sale entre.

4. Canule (1) selon l'une quelconque des revendications précédentes, **caractérisée en ce que** ledit corps (2) comprend deux indicateurs (4) situés aux parties supérieure et inférieure des ouvertures veineuses supérieures (6).

5. Canule (1) selon l'une quelconque des revendications précédentes, **caractérisée en ce que** ladite ouverture artérielle (7) par laquelle le sang propre est éjecté est située sur le corps (2) sous l'indicateur (4) situé sous l'ouverture veineuse supérieure (6).

6. Canule (1) selon l'une quelconque des revendications précédentes, **caractérisée par** :
- des indicateurs (4) situés en haut et en bas des ouvertures veineuses supérieures (6) en haut du corps (2), indiquant le début et la fin des ouvertures, et/ou
- un indicateur (4) situé juste au-dessus de l'extrémité inférieure de panier veineux (8) au bas du corps (2), qui est l'indicateur radio-opaque inférieur de la lumière veineuse et/ou
- un indicateur (4) qui est en forme de C et est situé sur l'ouverture (7) du corps (2) définissant l'ouverture artérielle.

7. Canule (1) selon l'une quelconque des revendications précédentes, **caractérisée en ce que** ladite ouverture veineuse supérieure (6) qui est située sur le haut du corps (2) est configurée de telle sorte que sa forme et son nombre varient en fonction de la taille du patient et est utilisée pour l'introduction de sang contaminé.

8. Canule selon la revendication 7, **caractérisée en ce que** ladite canule (1) comprend plus d'une ouverture veineuse supérieure (6) à section ronde entre les deux indicateurs (4) situés sur la partie supérieure du corps (2) lorsque la taille du patient augmente.

9. Canule (1) selon la revendication 7 ou 8, **caractérisée en ce que** ladite canule (1) comprend des ouvertures veineuses supérieures (6) qui sont situées progressivement sur le corps (2).

10. Canule (1) selon la revendication 7, **caractérisée en ce que** ladite canule (1) comprend des ouvertures veineuses supérieures à section elliptique entre les deux indicateurs (4) situés sur la partie supérieure du corps où la taille du patient est réduite.

11. Canule selon l'une quelconque des revendications précédentes, **caractérisée en ce que** ladite ouverture artérielle (7) a une section ronde ou transversale ou elliptique longitudinale et à laquelle le sang propre est éjecté.

12. Canule (1) selon l'une quelconque des revendications précédentes, **caractérisée en ce que** ladite extrémité inférieure de panier veineux (8) a plus d'un trou appelé trous de l'extrémité inférieure (10) sur sa partie supérieure.

13. Canule (1) selon la revendication 12, **caractérisée en ce que** ladite canule comprend des ouvertures d'extrémité de panier (9) situées à l'extrémité distale de la lumière veineuse.

14. Canule (1) selon l'une quelconque des revendications précédentes, dans laquelle ledit trou d'extrémité inférieur (10) situé sous le corps (2) et au-dessus de l'extrémité veineuse inférieure (8) est configuré de telle sorte que son nombre et sa forme varient en fonction de la taille du patient de telle sorte que ;
- lorsque la taille du patient est agrandie, il y a une pluralité de trous d'extrémité inférieurs (10) à section ronde ou elliptique sur le haut de l'extrémité inférieure de panier veineux (8) en dessous du corps (2), ou
- lorsque la taille du patient est plus petite, il y a des trous d'extrémité inférieure (10) de forme elliptique ou ronde sur le haut de l'extrémité inférieure de panier veineux (8) sous le corps (2).

15. Canule (1) selon l'une quelconque des revendications précédentes, **caractérisée en ce que** ladite ouverture veineuse supérieure (6) et ledit trou d'extrémité inférieur (10) sont situés des deux côtés du corps (2) afin de fournir une augmentation de la surface pour l'aspiration du sang de telle sorte qu'il y aura plus de puissance d'aspiration avec un corps étroit (2).
